# EUROPEAN PATENT SPECIFICATION

(11) **EP 1 003 528 B1**
(45) Date of publication and mention of the grant of the patent: **25.09.2002**
(21) Application number: 98935892.4
(22) Date of filing: 21.07.1998
(51) Int. Cl.: A61K 31/75, A61K 31/525, A61P 11/06, A61P 37/08

(54) **METHOD OF ENHANCING BIOAVAILABILITY OF FEXOFENADINE AND ITS DERIVATIVES**
VERFAHREN ZUR VERBESSERUNG DER BIOVERFÜGBARKEIT DES FEXOFENADINS UND SEINER DERIVATE
PROCEDE PERMETTANT DE RENFORCER LA BIODISPONIBILITE DE LA FEXOFENADINE ET SES DERIVES

(30) Priority: 14.08.1997 US 911563
(43) Date of publication of application: 31.05.2000
(73) Proprietor: Aventis Pharmaceuticals Inc., Bridgewater, NJ 08807-0800 (US)
(72) Inventor: HWANG, Kin-Kai, Belle Mead, NJ 08502 (US); GIESING, Dennis, H., New Jersey 08833, (US); HURST, Gail, H., Stilwell, KS 66085 (US)
(74) Representative: Minoja, Fabrizio, Dr.
(86) International application number: US9815098
(87) International publication number: WO99008690

(56) References cited:
- WO-A-95/20980
- WO-A-96/40192

## Description

### Background

The term "multidrug resistance" (MDR) describes the phenomenon whereby certain cancerous tumor cells develop a resistance to broad classes of cytotoxic agents when exposed to an individual cytotoxic agent. In other words, after a certain period of treatment with a cytotoxic agent which initially shows efficacy in controlling the growth of the tumor, the tumor develops a resistance not only to the specific agent to which the tumor was exposed, but also to broad classes of structurally and functionally unrelated agents. It has recently been found that MDR tumor cells over express a particular membrane glycoprotein known as p-glycoprotein ("p" for permeability). This p-glycoprotein is a member of the superfamily of ATP-binding cassette (ABC) transporters. It is thought that the exposure of the MDR tumor cells to a cytotoxic agent causes the induction of this p-glycoprotein which mediates a reverse transport system located on the tumor cell membrane that pumps the cytotoxic agent, as well as the other broad classes of cytotoxic agents, out of the tumor cell thus providing mutiple drug resistance for the cell.

P-glycoprotein is not just found in tumor cells. It is also expressed in a variety of normal, non-cancerous, epithelial and endothelial cells including in such tissues as the adreneal cortex, in the brush border of the proximal renal tubule epithelium, on the lumenal surface of biliary hepatocytes, in pancreatic ductules, and in the mucosa of the small and large intestine. For purposes of describing the present invention, the presence of p-glycoprotein in the small and large intestine is of particular interest.

When substances are ingested, they are mixed with digestive substances secreted by the body and are ultimately combined in a mixture in the lumen of the intestine. The lumen of the intestine is in contact with certain special epithelial cells which form the mucosa of the intestine or the intestinal wall. Nutrients and other substances present in the intestinal lumen passively diffuse into these intestinal epithelial cells and later diffuse into the portal circulation which carries the nutrients via the blood stream on to the liver. Thus, nutrients and other substances are absorbed into the body and become bioavailable for use by other tissues in the body.

The intestinal epithelial cells, however, do not just operate as a vehicle for passive diffusion of nutrients and other ingested substances. In addition, there are various active transport mechanisms located in the outer membrane of the epithelial cells which actively transport various nutrients and other substances into the cell. It is now thought that one of the active transport mechanisms present in the intestinal epithelial cells is p-glycoprotein transport mechanism which facilitates the reverse transport of substances, which have diffused or have been transported inside the cell, back into the lumen of the intestine. It has been speculated that the p-glycoprotein present in the intestinal epithelial cells may function as a protective reverse pump which prevents toxic substances which have been ingested and diffused or transported into the epithelial cell from being absorbed into the circulatory system and becoming bioavailable. One of the unfortunate aspects of the function of the p-glycoprotein in the intestinal cell however is that it can also function to prevent bioavailability of substances which are beneficial, such as certain drugs which happen to be substates for the p-glycoprotein reverse transport system.

It has now been found that, surprisingly, the antihistamines of the present invention are coincidentally also targeted by the p-glycoprotein reverse transport system in intestinal epiothelial cells and therefore are not fully bioavailable. The present invention successfully provides a composition for enhancing the bioavailablilty of these antihistamines.

### Summary of the Invention

The present invention relates to the use of a composition in the manufacture of a medicament for enhancing the bioavailability of a piperidinoalkanol antihistamine of Formula I 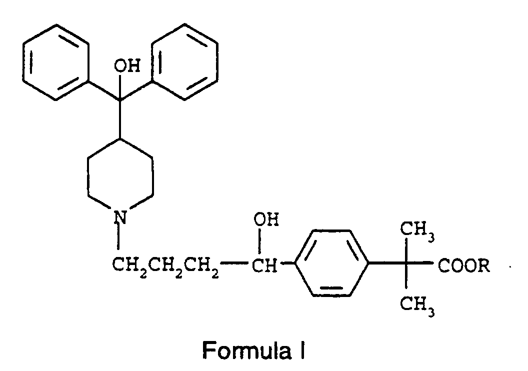 wherein R is hydrogen or C₁-C₆ alkyl,
or a pharmaceutically acceptable salt or an individual optical isomer thereof, in a patient wherein a co-administerable effective antihistaminic amount of said piperidinoalkanol and an effective p-glycoprotein inhibiting amount of a p-glycoprotein inhibitor are useful. The present invention further relates to a composition for treating allergic reactions in a patient, which comprises coadministering to said patient an effective antihistaminic amount of a piperidinoalkanol antihistamine of Formula I and an effective p-glycoprotein inhibiting amount of a p-glycoprotein inhibitor. The present invention also relates to a pharmaceutical composition comprising an effective antihistaminic amount of a piperidinoalkanol antihistamine of Formula I and an effective p-glycoprotein inhibiting amount of a p-glycoprotein inhibitor.

### Detailed Description of the Invention

The present invention provides a composition for enhancing bioavailability of a piperidinoalkanol antihistamine of Formula I 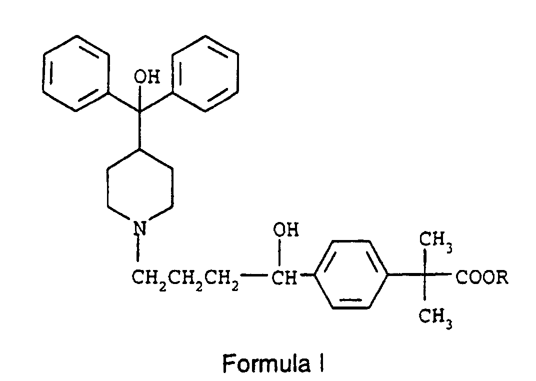 wherein R is hydrogen or C₁-C₆ alkyl,
or a pharmaceutically acceptable salt or an individual optical isomer thereof.

As used herein, the term "C₁-C₆ alkyl" refers to a saturated hydrocarbyl radical of straight or branched chain configuration of from 1 to 6 carbon atoms. Specifically included within the scope of the term "C₁-C₆ alkyl" are the hydrocarbyl radicals methyl, ethyl, propyl, isopropyl, butyl, sec-butyl, isobutyl, tert-butyl, pentyl, hexyl, and the like. One skilled in the art would immediately recognize and appreciate that the compounds of Formula I possess a chiral center and as such exist in stereoisomeric forms. The present invention applies to the racemic mixture of these stereoisomeric forms as well as to the isolated individual stereoisomers. The individual stereoisomers can be isolated from the racemic mixture by separation techniques which are well known and appreciated in the art including chromatographic methods and selective crystallization techniques.

The compounds of Formula I may exist in their free form or as pharmaceutically acceptable salts. Pharmaceutically acceptable salts of the compounds of Formula I are those of any suitable inorganic or organic acid. Examples of suitable inorganic acids include hydrochloric, hydrobromic, sulfuric, and phosphoric acids. Examples of suitable organic acids include carboxylic acids, such as, acetic, propionic, glycolic, lactic, pyruvic, malonic, succinic, fumaric, malic, tartaric, citric, cyclamic, ascorbic, maleic, hydroxymaleic, dihydroxymaleic, benzoic, phenylacetic, 4-aminobenzoic, 4-hydroxybenzoic, anthranillic, cinnamic, salicylic, 4-aminosalicylic, 2-phenoxybenzoic, 2-acetoxybenzoic, mandelic acid, and sulfonic acids, such as, methanesulfonic, ethanesulfonic, and β-hydroxyethanesulfonic acid. Non-toxic salts of the compounds of Formula I formed with inorganic or organic bases are also included within the scope of this invention and include, for example, those of alkali earth metals, for example, calcium and magnesium, light metals of group IIIA, for example, aluminum, organic amines, such as, primary, secondary or tertiary amines, for example, cyclohexylamine, ethylamine, pyridine, methylaminoethanol, and piperazine. The salts of compounds of Formula I may be prepared by conventional means as, for example, by treating a compound of Formula I with an appropriate acid or base. The preferred pharmaceutically acceptable salt for compounds of Formula I is the hydrochloric acid salt.

Compounds of Formula I may be prepared as described in U.S. Patent No. 4,254,129.

The preferred compound of Formula I is the compound (±)-4-[1-hydroxy-4-[4-(hydroxydiphenylmethyl)-1-piperidinyl]-butyl]-α,α-dimethyl benzeneacetic acid, which is also known as fexofenadine, and its individual stereoisomers. Fexofenadine, as the hydrochloric acid salt, has been recently approved by the United States Food and Drug Administration (FDA) for use as the active ingredient in the antihistamine known as Allegra™. Allegra is indicated for the treatment of seasonal allergic rhinitis with recommended dosing at 60mg B.I.D.

The present invention provides a composition for enhancing bioavailability of the compounds of Formula I. The co-administration of an effective antihistaminic amount of a compound of Fromula I along with an effective p-glycoprotein inhibiting amount of a p-glycoprotein inhibitor provides an enhanced bioavailability for the compounds of Formula I. Bioavailability of a drug is defined as the degree to which a drug becomes available to the target tissue after administration and is conveniently measured as the total amount of drug available systemically. Typically, bioavailability is assessed by measuring the drug concentration in the blood at various points of time after administration of the drug and then integrating the values obtained over time to yield the total amount of drug circulating in the blood. This measurement, called the Area Under the Curve (AUC), is a direct measurement of the bioavailability of the drug. Alternatively, bioavailability may be assessed for fexofenadine by measuring total urine output of fexofenadine, since it is known that fexofenadine is not significantly metabolized after oral administration.

The present invention relates to an enhancement of the bioavailability of the drug of Formula I by co-administration of a p-glycoprotein inhibitor. By co-administration of a compound of Formula I and a p-glycoprotein inhibitor, the total amount of the compound of Formula I is increased over that which would otherwise circulate in the blood in the absence of the p-glycoprotein inhibitor. Thus, co-administration will cause an increase in the AUC of the compound of Formula I over that seen with administration of the compound of Formula I alone.

As used herein, the term "patient" refers to a mammal, such as, for example, a human, mouse, rat, dog, cat, and the like, which is in need of treatment for an allergic reaction. As used herein, the term "allergic reaction" refers to a histamine-mediated allergic disease, such as, for example, seasonal allergic rhinitis, idiopathic urticaria, and the like. Such diseases are generally distinguished by an allergen triggered release of histamine from storage cells in tissues. The released histamine binds certain H₁-histamine receptors which results in the manifestation of the well known allergic symptioms such as sneezing, itching skin, itching eyes, rhinorrhea, etc. An antihistamine, such as the compounds of Formula I, will block manifestation of the allergic symptoms caused by release of histamine by blocking the H₁-histamine receptors in various tissues in the body, such as in the skin, lungs or the nasal mucosa. Antihistamines, such as the compounds of Formula I, are thus well known and effective treatment for allergic reactions in patients.

Enhancement of bioavailability of a compound of Formula I will provide for a more efficient and effective treatment of the patient since, for a given dose, more compound will be available at the tissue sites at which the antihistamine blocks H₁-histamine receptors than in the absence of this enhanced bioavailability.

Administration of the compound of Formula I refers to oral administration. The compound of Formula I may be administered orally in any convenient dosage form including, for example, capsule, tablet, liquid, suspension, and the like.

An effective antihistaminic amount of a compound of Formula I is that amount which is effective in providing an antihistaminic effect in a patient. An effective antihistaminic amount will vary between about 1mg to about 600mg of a compound of Formula I as a daily dose depending upon the type of disease to be treated, the degree of severity of the disease, the species of patient to be treated, the dosage regimen, and other factors which are all well within the abilities of one of ordinary skill in the medical arts to evaluate and assess. A preferred amount however will typically be from about 10mg to about 240mg, a more preferred amount will typically be from about 20mg to about 180mg, and a further preferred amount will typically be from about 40mg to about 120mg. The most preferred amount of a compound of Formula I will be 60mg or 120mg. The above amounts of a compound of Formula I can be administered from once to multiple times per day. Typically, doses will be administered on a regimen requiring one, two or three doses per day with one and two being the preferred. The more preferred doseage and regimen will be 40mg twice per day, 60mg twice per day, 80mg twice per day, 80mg once daily, 120mg once daily, and 180mg once daily with the most preferred being 60mg twice per day and 120mg once daily.

As used herein, the term "p-glycoprotein inhibitor" refers to organic compounds which inhibit the activity of the p-glycoprotein mediated active transport system present in the gut. This transport system actively transports drugs which have been absorbed from the intestinal lumen and into the gut epithelium back out into the lumen. Inhibition of this p-glycoprotein mediated active transport system will cause less drug to be transported back into the lumen and will thus increase the net drug transport across the gut epithelium and will increase the amount of drug ultimately available in the blood.

Various p-glycoprotein inhibitors are well known and appreciated in the art. These include, water soluble vitamin E; polyethylene glycol; poloxamers including Pluronic F-68; Polyethylene oxide; polyoxyethylene castor oil derivatives including Cremophor EL and Cremophor RH 40; Chrysin, (+)-Taxifolin; Naringenin; Diosmin; Quercetin; and the like.

Polyethylene glycols (PEGs) are liquid and solid polymers of the general formula H(OCH₂CH₂)ₙOH, where n is greater than or equal to 4, having various average molecular weights ranging from about 200 to about 20000. PEGs are also known as α-hydro-ω-hydroxypoly-(oxy-1,2-ethanediyl)polyethylene glycols. For example, PEG 200 is a polyethylene glycol wherein the average value of n is 4 and the average molecular weight is from about 190 to about 210. PEG 400 is a polyethylene glycol wherein the average value of n is between 8.2 and 9.1 and the average molecular weight is from about 380 to about 420. Likewise, PEG 600, PEG 1500 and PEG 4000 have average values of n of 12.5-13.9, 29-36 and 68-84, respectively, and average molecular weights of 570-630, 1300-1600 and 3000-3700, respectively, and PEG 1000, PEG 6000 and PEG 8000 have average molecular weights of 950-1050, 5400-6600, and 7000-9000, respectively. Polyethylene glycols of varying average molecular weight of from 200 to 20000 are well known and appreciated in the art of pharmaceutical science and are readily available.

The preferred polyethylene glycols for use in the instant invention are polyethylene glycols having an average molecular weight of from about 200 to about 20,000. The more preferred polyethylene glycols have an average molecular weight of from about 200 to about 8000. More specifically, the more preferred polyethylene glycols for use in the present invention are PEG 200, PEG 400, PEG 600, PEG 1000, PEG 1450, PEG 1500, PEG 4000, PEG 4600, and PEG 8000. The most preferred polyethylene glycols for use in the instant invention is PEG 400, PEG 1000, PEG 1450, PEG 4600 and PEG 8000.

Polysorbate 80 is an oleate ester of sorbitol and its anhydrides copolymerized with approximately 20 moles of ethylene oxide for each mole of sorbitol and sorbitol anhydrides. Polysorbate 80 is made up of sorbitan mono-9-octadecanoate poly(oxy-1,2-ethandiyl) derivatives. Polysorbate 80, also known as Tween 80®, is well known and appreciated in the pharmaceutical arts and is readily available.

Water-soluble vitamin E, also known as d-α-tocopheryl polyethylene glycol 1000 succinate [TPGS], is a water-soluble derivative of natural-source vitamin E. TPGS may be prepared by the esterification of the acid group of crystalline d-α-tocopheryl acid succinate by polyethylene glycol 1000. This product is well known and appreciated in the pharmaceutical arts and is readily available. For example, a water-soluble vitamin E product is available commercially from Eastman Corporation as Vitamin E TPGS®.

Naringenin is the bioflavonoid compound 2,3-dihydro-5,7-dihydroxy-2-(4-hydroxyphenyl)-4H-1-benzopyran-4-one and is also known as 4',5,7-trihydroxyflavanone. Naringenin is the aglucon of naringen which is a natural product found in the fruit and rind of grapefruit. Naringenin is readily available to the public from commercial sources.

Quercetin is the bioflavonoid compound 2-(3,4-dihydroxyphenyl)-3,5,7-trihydroxy-4H-1-benzopyran-4-one and is also known as 3,3',4',5,7-pentahydroxyflavone. Quercetin is the aglucon of quercitrin, of rutin and of other glycosides. Quercetin is readily available to the public from commercial sources.

Diosmin is the naturally occurring flavonic glycoside compound 7-[[6-O-6-deoxy-α-L-mannopyranosyl)-β-D-glucopyranosyl]oxy]-5-hydroxy-2-(3-hydroxy-4-methoxyphenyl)-4H-1-benzopyran-4-one. Diosmin can be isolated from various plant sources including citrus fruits. Diosmin is readily available to the public from commercial sources.

Chrysin is the naturally occurring compound 5,7-dihydroxy-2-phenyl-4H-1-benzopyran-4-one which can be isolated from various plant sources. Chrysin is readily available to the public from commercial sources.

Poloxamers are α-hydro-ω-hydroxypoly(oxyethylene)poly(oxypropylene)-poly(oxyethylene) block copolymers. Poloxamers are a series of closely related block copolymers of ethylene oxide and propylene oxide conforming to the general formula HO(C₂H₄O)ₐ(C₃H₆O)_{b}(C₂H₄O)ₐH. For example, poloxamer 124 is a liquid with "a" being 12, "b" being 20, and having an average molecular weight of from about 2090 to about 2360; poloxamer 188 is a solid with "a" being 80, "b" being 27, and having an average molecular weight of from about 7680 to 9510; poloxamer 237 is a solid with "a" being 64, "b" being 37, and having an average molecular weight of from about 6840 to 8830; poloxamer 338 is a solid with "a" being 141, "b" being 44, and having an average molecular weight of from about 12700 to 17400; and poloxamer 407 is a solid with "a" being 101, "b" being 56, and having an average molecular weight of from about 9840 to 14600. Poloxamers are well known and appreciated in the pharmaceutical arts and are readily available commercially. For example, Pluronic F-68 is a commercially available poloxamer from BASF Corp. The preferred poloxamers for use in the present invention are those such as poloxamer 188®, Pluronic F-68®.

Polyoxyethylene castor oil derivatives are a series of materials obtained by reacting varying amounts of ethylene oxide with either castor oil or hydrogenated castor oil. These polyoxyethylene castor oil derivatives are well known and appreciated in the pharmaceutical arts and several different types of material are commercially available, including the Cremophors available from BASF Corporation. Polyoxyethylene castor oil derivatives are complex mixtures of various hydrophobic and hydrophilic components. For example, in polyoxyl 35 castor oil (also known as Cremophor EL), the hydrophobic constituents comprise about 83% of the total mixture, the main component being glycerol polyethylene glycol ricinoleate. Other hydrophobic constituents include fatty acid esters of polyethylene glycol along with some unchanged castor oil. The hydrophilic part of polyoxyl 35 castor oil (17%) consists of polyethylene glycols and glyceryl ethoxylates.

In polyoxyl 40 hydrogenated castor oil (Cremophor RH 40®) approximately 75% of the components of the mixture are hydrophobic. These comprise mainly fatty acid esters of glycerol polyethylene glycol and fatty acid esters of polyethylene glycol. The hydrophilic portion consists of polyethylene glycols and glycerol ethoxylates. The preferred polyoxyethylene castor oil derivatives for use in the present invention are polyoxyl 35 castor oil, such as Cremophor EL®, and polyoxyl 40 hydrogenated castor oil, such as Cremophor RH 40®. Cremophor EL® and Cremophor RH® 40 are commercially available from BASF Corporation.

Polyethylene oxide is a nonionic homopolymer of ethylene oxide conforming to the general formula (OCH₂CH₂)ₙ in which n represents the average number of oxyethylene groups. Polyethylene oxides are available in various grades which are well known and appreciated by those in the pharmaceutical arts and several different types of material are commercially available. The preferred grade of polyethylene oxide is NF and the like which are commercially available.

(+)-Taxifolin is (2R-trans)-2-(3,4-dihydroxyphenyl)-2,3-dihydro-3,5,7-trihydroxy-4H-1-benzopyran-4-one. Other common names for (+)-taxifolin are (+)-dihydroquercetin; 3,3',4',5,7-pentahydroxy-flavanone; diquertin; taxifoliol; and distylin. (+)-Taxifolin is well know and appreciated in the art of pharmaceutical arts and is readily available commercially.

The preferred p-glycoprotein inhibitor for use in the present invention are water soluble vitamin E, such as vitamin E TPGS®, and the polyethylene glycols. Of the polyethylene glycols, the most preferred p-glycoprotein inhibitors are PEG 400, PEG 1000, PEG 1450, PEG 4600 and PEG 8000.

Administration of a p-glycoprotein inhibitor may be by any route by which the p-glycoprotein inhibitor will be bioavailable in effective amounts including oral and parenteral routes. Although oral administration is preferred, the p-glycoprotein inhibitors may also be administered intravenously, topically, subcutaneously, intranasally, rectally, intramuscularly, or by other parenteral routes. When administered orally, the p-glycoprotein inhibitor may be administered in any convenient dosage form including, for example, capsule, tablet, liquid, suspension, and the like.

An effective p-glycoprotein inhibiting amount of a p-glycoprotein inhibitor is that amount which is effective in providing inhibition of the activity of the p-glycoprotein mediated active transport system present in the gut. An effective p-glycoprotein inhibiting amount will vary between about 5 mg to about 1000 mg of p-glycoprotein inhibitor as a daily dose depending upon the particular p-glycoprotein inhibitor selected, the species of patient to be treated, the dosage regimen, and other factors which are all well within the abilities of one of ordinary skill in the medical arts to evaluate and assess. A preferred amount however will typically be from about 50 mg to 500 mg, and a more preferred amount will typically be from about 100 mg to 500 mg. The above amounts of a p-glycoprotein inhibitor can be administered from once to multiple times per day. Typically for oral dosing, doses will be administered on a regimen requiring one, two or three doses per day with one and two being the preferred.

Where water soluble vitamin E or a polyethylene glycol is selected as the p-glycoprotein inhibitor, a preferred amount will typically be from about 5 mg to 1000 mg, a more preferred amount will typically be from about 50 mg to 500 mg, and a further preferred amount will typically be from about 100 mg to 500 mg. The most preferred amount of water soluble vitamin E or a polyethylene glycol will be from about 200 mg to 500 mg. The above amounts of water soluble vitamin E or polyethylene glycol can be administered from once to multiple times per day. Typically, doses will be administered on a regimen requiring one, two or three doses per day with one and two being preferred.

As used herein, the term "co-administration" refers to administration to a patient of both a compound of Formula I and a p-glycoprotein inhibitor so that the pharmacologic effect of the p-glycoprotein inhibitor in inhibiting p-glycoprotein mediated transport in the gut is manifest at the time at which the compound of Formula I is being absorbed from the gut. Of course, the compound of Formula I and the p-glycoprotein inhibitor may be administered at different times or concurrently. For example, the p-glycoprotein inhibitor may be administered to the patient at a time prior to administration of the compound of Formula I so as to pre-treat the patient in preparation for dosing with the compound of Formula I. Furthermore, it may be convenient for a patient to be pre-treated with the p-glycoprotein inhibitor so as to achieve steady state levels of p-glycoprotein inhibitor prior to administration of the first dose of the compound of Formula I. It is also contemplated that the compound of Formula I and the p-glycoprotein inhibitor may be administered essentially concurrently either in separate dosage forms or in the same oral dosage form.

The compound of Formula I and the p-glycoprotein inhibitor may be administered in separate dosage forms or in the same combination oral dosage form. Co-administration of the compound of Formula I and the p-glycoprotein inhibitor may conveniently be accomplished by oral administration of a combination dosage form containing both the compound of Formula I and the p-glycoprotein inhibitor.

Thus, an additional embodiment of the present invention is a combination pharmaceutical composition for oral administration comprising an effective antihistaminic amount of a compound of Formula I (the antihistamine) and an effective p-glycoprotein inhibiting amount of a p-glycoprotein inhibitor (the inhibitor). This combination oral dosage form may provide for immediate release of both the compound of Formula I and the p-glycoprotein inhibitor or may provide for sustained release of one or both of the compound of Formula I and the p-glycoprotein inhibitor. One skilled in the art would readily be able to determine the appropriate properties of the combination dosage form so as to achieve the desired effect of co-administration of the compound of Formula I and the p-glycoprotein inhibitor.

The antihistamine and the inhibitor may be administered alone or in the form of a pharmaceutical composition in admixture or otherwise in association with one or more pharmaceutically acceptable carriers or excipients, the proportion and nature of which are determined by the solubility and chemical properties of the antihistamine and inhibitior selected, the dosage regimen desired and standard pharmaceutical practice. The antihistamines, while effective themselves, may be formulated and administered in the form of their pharmaceutically acceptable acid addition salts, such as the hydrochloride, for purposes of stability, convenience of cystallization, increased solubility. One form of the pharmaceutical composition according to the present invention is a combination pharmaceutical composition where both the antihistamine and the inhibitor are present in the same dosage form.

The pharmaceutical composition may be prepared in a manner well known and appreciated in the pharmaceutical art. The carrier or excipient is pharmacologically inert and may be a solid, semi-solid, or liquid material which can serve as a vehicle or medium for the antihistamine and the inhibitor. Suitable carriers and excipients are well known in the art. The pharmaceutical compositon may be adapted for oral administration in the form of a tablet, capsule, liquid, syrup, wafer, chewing gum, suspension, or the like. These preparations may contain at least 4% of active ingredient, i.e., the percent by weight of the antihistamine and the inhibitor, but may conveniently be varied depending upon the particular form so that the active ingredients make up from about 4% to 70% of the weight of the unit dosage form.

Tablets, pills, capsules, and the like may contain one or more of the following carriers or excipients: binders such as microcrystalline cellulose, gum tragacanth or gelatin; excipients such as starch or lactose; surfactants such as polysorbate 80, and the like; disintegrating agents such as alginic acid, primogel™, com starch, sodium bicarbonate, calcium bicarbonate and the like; lubricants such as magnesium stearate or Sterotex™; glidants such as colloidal silicon dioxide; sweetening agents such as sucrose or saccharin; flavoring agent such as peppermint, methyl salicylate or orange flavoring. Capsules may contain, in addition to the ingredients listed above for tablets, a liquid carrier such as polyethylene glycol or a fatty oil. Tablets and capsules may contain other various carriers and excipients which modify the physical form of the dosage unit, for example, as coatings. Thus, tablets may be coated with sugar, shellac, or other enteric coating agents. A syrup may contain, in addition to the active ingredients, sterile water, sucrose as a sweetening agent, preservatives, dyes, and colorings and flavors. Materials used in preparing these various compositions should be pharmaceutically pure and non-toxic in the amounts used.

For purposes of parenteral administration, the inhibitor may be incorporated into a solution or suspension. These preparations should contain at least 0.1% of the active ingredient but may be varied from about 0.1% to 50% by weight thereof. The amount of the inhibitor should be adjusted in such compositions so that an a suitable dosage will be obtained upon administration.

The solutions or suspensions may also include one or more of the following adjuvants: sterile diluents such as water, saline, fixed oils, polyethylene glycols, glycerine, propylene glycols or other synthetic solvents; antibacterial agents such as benzyl alcohol or methyl paraben; antioxidants such as ascorbic acid or sodium bisulfite; chelating agents such as ethylene diaminetetraacetic acid; buffers such as acetates, citrates or phosphates; agents for the adjustment of tonicity such as sodium chloride or dextrose. The parenteral preparations may be enclosed in ampules, disposable syringes or multiple dosage vials made of glass or plastic.

More particularly, the combination pharmaceutical composition may be in the form of a tablet, a capsule, a liquid, a suspension, a syrup, and the like. The combination pharmaceutical composition, including in tablet form, may be a simple admixture of the antihistamine, the inhibitor, and any necessary and appropriate carriers and excipients. Alternatively, the composition may be in the form of an admixture of various heterogeneous pellets, beads or other heterogeneous particles which provide an appropriate formulation. In addition, the pharmaceutical composition may be in the form of a multiple compression tablet such as a multilayered tablet or a compression-coated tablet.

Combination pharmaceutical compositions made up of heterogeneous pellets, beads or particles (hereinafter referred to as "heterogeneous pellets"), or made up of multiple compression tablets, are useful for administration of pharmaceutical compositions which provide for different release characteristics for the antihistamine and inhibitor. For example, these compositions may provide for an immediate release of the inhibitor and a sustained release of the antihistamine, or vice versa. These compositions are prepared according to standard techniques which are well known and appreciated in the art such as those described in U.S. Patent No. 4,996,061

The following examples illustrate a particularly preferred embodiment of the present invention. These examples are illustrative only and are not intended to limit the scope of the invention in any way.

### EXAMPLE 1

### Effect of PEG 400 on the Bioavailabilty of Fexofenadine in the Dog

The effect of polyethylene glycol 400 (PEG 400) on the bioavailability of fexofenadine was determined in two fasted, male beagle dogs. Treatment A consisted of oral administration of one 120 mg fexofenadine hydrochloride sustained release (SR) tablet, and treatment B consisted of oral administration of one SR tablet together with a capsule with 0.5 mL PEG 400 given at -1, 0, 2, 4, 6, and 8 hours before and after the SR tablet. Treatment A was given 10 or 17 days prior to Treatment B. The plasma concentrations of fexofenadine were analyzed to determine relative bioavailability of fexofenadine with and without concomitant treatment with PEG 400.

A mean 2-fold increase in plasma concentrations (Table I) occurred when PEG 400 was co-administered with fexofenadine. This doubling of fexofenadine bioavailability is also shown in Fiqure 1, which illustrates the increase in mean plasma concentrations produced during co-administration.

**Table I**

| Plasma Concentrations of Fexofenadine in Dogs Given a 120 mg Fexofenadine SR Tablet Dose Alone or with 0.5 mL PEG-400 Capsule Doses | | | | |
|---|---|---|---|---|
| | | Fexofenadine | Concentration | (ng/mL) |
| Dose Condition | Time (Hours) | Dog | Number | |
| | | 7645 | 3181 | Mean |
| | | | | |
| Fexofenadine Alone | 0 | 0 | 0 | 0 |
| | 0.5 | 192.93 | 221.88 | 207.41 |
| | 1 | 523.96 | 1196.64 | 860.30 |
| | 1.5 | 748.57 | 1537.07 | 1142.82 |
| | 2 | 1617.8 | 2088.09 | 1852.95 |
| | 3 | 2316.21 | 1865.81 | 2091.01 |
| | 5 | 2364.18 | 793.03 | 1578.61 |
| | 7 | 1170.93 | 276.88 | 723.91 |
| | 9 | 880.07 | 184.32 | 532.20 |
| | 12 | 350.02 | 91.25 | 220.64 |
| | 14 | 274.33 | 69.49 | 171.91 |
| | 22 | 110.33 | 28.95 | 69.64 |
| | 24 | 97.87 | 34.68 | 66.28 |
| | | | | |
| Fexofenadine + | | | | |
| PEG-400 | 0 | 0 | 0 | 0 |
| | 0.5 | 783.93 | 154.38 | 469.16 |
| | 1 | 5866.28 | 687.3 | 3276.79 |
| | 1.5 | 7574.3 | 820.16 | 4197.23 |
| | 2 | 10116.53 | 1277.5 | 5697.02 |
| | 3 | 9794.6 | 3736.69 | 6765.65 |
| | 5 | 4794.46 | 1342.66 | 3068.56 |
| | 7 | 1400.87 | 565.14 | 983.01 |
| | 9 | 890.27 | 240.76 | 565.52 |
| | 12 | 585.41 | 139.86 | 362.64 |
| | 14 | 293.91 | 82.72 | 188.32 |
| | 22 | 108.74 | 59.66 | 84.20 |
| | 24 | 93.73 | 51.54 | 72.64 |

### EXAMPLE 2

Effect of Water Suluble Vitamin E on the Bioavailability of Fexofenadine in the Dog

The effect of water soluble vitamin E (d-α-tocopheryl polyethylene glycol succinate) on the bioavailability of fexofenadine was determined in two fasted, male beagle dogs in a two-way crossover experimental design. Treatment A consisted of oral administration of an aqueous solution of a 1 mg/kg dose of ¹⁴C-labeled fexofenadine alone, and Treatment B consisted of oral administration of an aqueous solution of the same dose of ¹⁴C-labeled fexofenadine and a 10 IU/Kg dose of water soluble vitamin E. Treatments were given in the opposing order of a crossover design in the two dogs, and a one week washout period occurred between treatments. The radioactivity in plasma and urine was analyzed and is known to represent unchanged fexofenadine in the dog.

The results showed a 50% increase in plasma ¹⁴C AUC occurred when water soluble vitamin E was co-administered with ¹⁴C fexofenadine (Table II). That is, the bioavailability of fexofenadine was increased 50% by water soluble vitamin E. Figure 2 illustrates the increase in mean plasma concentrations caused by co-administration of water soluble vitamin E.

**Table II**

| Plasma Concentrations of [¹⁴C]Fexofenadine in Dogs Given a 1 mg/kg [¹⁴C]Fexofenadine Oral Solution Dose Alone or with 10 IU/kg Water Soluble Vitamin E | | | | |
|---|---|---|---|---|
| | | [¹⁴C]Fexofenadine | Concentration | (ng equiv/mL) |
| Dose Condition | Time (Hours) | Dog | Number | |
| | | 7645 | 3181 | Mean |
| | | | | |
| Fexofenadine Alone | 0 | 0 | 0 | 0 |
| | 0.5 | 509 | 829 | 669 |
| | 1 | 546 | 673 | 609.5 |
| | 1.5 | 815 | 743 | 779 |
| | 2 | 924 | 559 | 741.5 |
| | 3 | 882 | 386 | 634 |
| | 5 | 330 | 128 | 229 |
| | 7 | 155 | 81 | 118 |
| | 9 | 82 | 54 | 68 |
| | 12 | 40 | 26 | 33 |
| | 14 | 33 | 18 | 25.5 |
| | 22 | 15 | 5 | 10 |
| | 24 | 9 | 8 | 8.5 |
| | | | | |
| Fexofenadine + | | | | |
| WS Vit E | 0 | 0 | 0 | 0 |
| | 0.5 | 853 | 1472 | 1162.5 |
| | 1 | 1721 | 1098 | 1409.5 |
| | 1.5 | 1974 | 805 | 1389.5 |
| | 2 | 1515 | 572 | 1043.5 |
| | 3 | 1104 | 558 | 831 |
| | 5 | 230 | 257 | 243.5 |
| | 7 | 163 | 120 | 141.5 |
| | 9 | 90 | 73 | 81.5 |
| | 12 | 51 | 40 | 45.5 |
| | 14 | 48 | 31 | 39.5 |
| | 22 | 14 | 11 | 12.5 |
| | 24 | 10 | 13 | 11.5 |

The increase in absorption and bioavailability of fexofenadine that occurred with concomitant administration of water soluble vitamin E was also evident from the urinary excretion of ¹⁴C fexofenadine in urine, which increased a mean of 3-fold (Table III).

**Table III**

| Percent of [¹⁴C]Fexofenadine Excreted in Urine of Dogs Given a 1 mg/kg Oral [¹⁴C]Fexofenadine Hydrochloride Dose Without or With Water Soluble Vitamin E Excipient. | | | |
|---|---|---|---|
| | Without Excipient | With Excipient | |
| Dog Number | (% Dose) | (%Dose) | Ratio |
| 7645 | 2.38 | 9.88 | 4.2 |
| 3181 | 2.80 | 4.79 | 1.7 |
| Mean | 2.59 | 7.34 | 3.0 |

### EXAMPLE 3

### Effect of PEG 1000 on the Bioavailabilty of Fexofenadine in the Dog

The effect of polyethylene glycol 1000 (PEG 1000) on the bioavailability of fexofenadine was determined in two fasted, male beagle dogs. Treatment A consisted of oral administration of one 120 mg fexofenadine hydrochloride sustained release (SR) tablet, and treatment B consisted of oral administration of one SR tablet together with a capsule containing 0.5 g PEG 1000 dissolved in 2.5 mL water given at -1, -0.1, and 4 hours before and after the SR tablet. Treatment A was given two months prior to Treatment B. The plasma concentrations of fexofenadine were analyzed to determine relative bioavailability of fexofenadine with and without concomitant treatment with PEG 1000.

A mean 2-fold increase in plasma concentrations [AUC(0-24h) values calculated from the concentrations shown in Table IV] occurred when PEG 1000 was co-administered with fexofenadine. The peak concentration was increased a mean of 3-fold. This increased bioavailability in the presence of PEG 1000 is evident in the graph of mean plasma fexofenadine concentrations (Figure 3).

**Table IV**

| Plasma Concentrations of Fexofenadine in Dogs Given a 120 mg Fexofenadine SR® Tablet Dose Alone or with 0.5 g PEG-1000 Capsule Solution Doses | | | | |
|---|---|---|---|---|
| | | Fexofenadine | Concentration | (ng/mL) |
| Dose Condition | Time (Hours) | Dog | Number | |
| | | 7645 | 3181 | Mean |
| Fexofenadine Alone | 0 | 0 | 0 | 0 |
| | 0.5 | 192.93 | 221.88 | 207.41 |
| | 1 | 523.96 | 1196.64 | 860.30 |
| | 1.5 | 748.57 | 1537.07 | 1142.82 |
| | 2 | 1617.8 | 2088.09 | 1852.95 |
| | 3 | 2316.21 | 1865.81 | 2091.01 |
| | 5 | 2364.18 | 793.03 | 1578.61 |
| | 7 | 1170.93 | 276.88 | 723.91 |
| | 9 | 880.07 | 184.32 | 532.20 |
| | 12 | 350.02 | 91.25 | 220.64 |
| | 14 | 274.33 | 69.49 | 171.91 |
| | 22 | 110.33 | 28.95 | 69.64 |
| | 24 | 97.87 | 34.68 | 66.28 |
| | | | | |
| Fexofenadine + | | | | |
| PEG-1000 | 0 | 0 | 0 | 0 |
| | 0.5 | 15.28 | 147.24 | 81.31 |
| | 1 | 669.27 | 473.48 | 571.38 |
| | 1.5 | 1133.02 | 1687.98 | 1410.50 |
| | 2 | 4541.31 | 3963.22 | 4252.27 |
| | 3 | 7695.42 | 5595.32 | 6645.37 |
| | 5 | 3398.34 | 2035.32 | 2716.83 |
| | 7 | 1320.73 | 857.89 | 1089.31 |
| | 9 | 784.42 | 377.1 | 580.76 |
| | 12 | 315.74 | 202.89 | 259.32 |
| | 24 | 109.69 | 112.75 | 111.22 |

## Claims

1. A combination for enhancing the bioavailability of a piperidinoalkanol antihistamine of the following Formula I 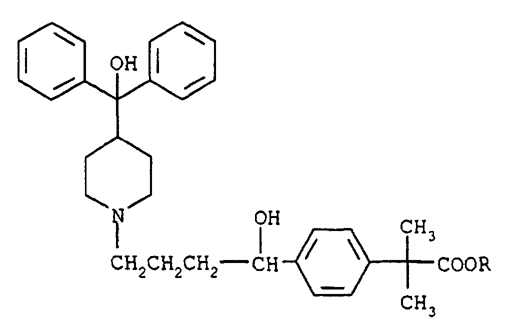 wherein R is hydrogen or C₁-C₆ alkyl,
or a pharmaceutically acceptable salt or an individual optical isomer thereof, which comprises an effective antihistaminic amount of said piperidinoalkanol antihistamine and an effective p-glycoprotein inhibiting amount of a p-glycoprotein inhibitor, in dosage form suitable for co-administration.

2. A combination according to claim 1 wherein the antihistamine is fexofenadine, or a pharmaceutically acceptable salt thereof.

3. A combination according to claim 1 or 2 wherein the p-glycoprotein inhibitor is selected from the group consisting of water soluble vitamin E and polyethylene glycols.

4. A combination according to any one of claims 1 to 3 wherein the p-glycoprotein inhibitor is water soluble vitamin E or is selected from the group consisting of PEG 400, PEG 1000, PEG 1450, PEG 4600 and PEG 8000.

5. A combination according to claim 4 wherein the p-glycoprotein inhibitor is water soluble vitamin E or PEG 1000.

6. A combination according to any one of claims 1 to 5 for use in the treatment of allergic reactions.

7. A combination according to any one of claims 1 to 6 wherein the p-glycoprotein inhibitor and the compound of Formula I are suitable for administration at different times or concurrently.

8. A combination according to any one of claims 1 to 7 wherein the p-glycoprotein inhibitor is suitable for administration at a time prior to the administration of the compound of Formula I.

9. A combination according to any one of claims 1 to 8 wherein the compound of Formula I and the p-glycoprotein inhibitor are suitable for essentially concurrent administration either in separate dosage form or in the same oral dosage form.

10. A combination according to claim 9 wherein the oral dosage form provides for immediate release of both the compound of Formula I and the p-glycoprotein inhibitor.

11. A combination according to claim 9 wherein the oral dosage form provides for sustained release of one or both of the compound of Formula I and the p-glycoprotein inhibitor.

12. A combination according to claim 11 wherein the dosage form is a multiple compression tablet.

13. A pharmaceutical composition comprising an effective antihistaminic amount of a piperidinoalkanol antihistamine of the following Formula I 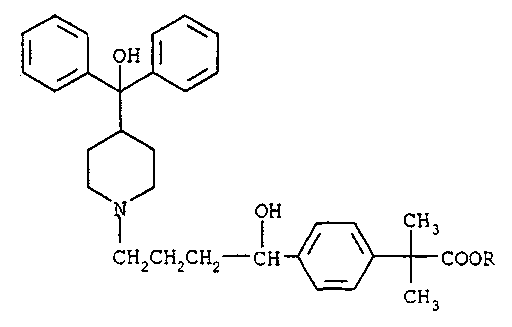 wherein R is hydrogen or C₁-C₆ alkyl,
or a pharmaceutically acceptable salt or an individual optical isomer thereof, and an effective p-glycoprotein inhibiting amount of a p-glycoprotein inhibitor.

14. A composition of claim 13 wherein the antihistamine is fexofenadine, or a pharmaceutically acceptable salt thereof.

15. A composition of claim 13 or 14 wherein the p-glycoprotein inhibitor is selected from the group consisting of water soluble vitamin E and polyethylene glycols.

16. A composition of any one of claims 13 to 15 wherein the p-glycoprotein inhibitor is water soluble vitamin E or is selected from the group consisting of PEG 400, PEG 1000, PEG 1450, PEG 4600 and PEG 8000.

17. A composition of any one of claims 13 to 16 wherein the p-glycoprotein inhibitor is water soluble vitamin E or PEG 1000.

18. The use of a composition in the manufacture of a medicament for enhancing bioavailability of a piperidinoalkanol antihistamine of the following Formula I 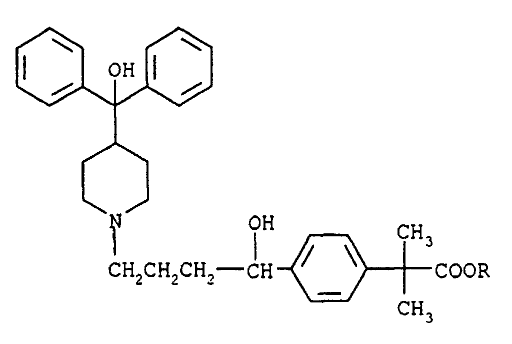 wherein R is hydrogen or C₁-C₆ alkyl,
or a pharmaceutically acceptable salt or an individual optical isomer thereof, wherein said composition comprises an effective antihistaminic amount of said piperidinoalkanol antihistamine and an effective p-glycoprotein inhibiting amount of a p-glycoprotein inhibitor.

19. Use according to Claim 18 wherein the antihistamine is fexofenadine, or a pharmaceutically acceptable salt thereof.

20. Use according to Claim 18 or 19 wherein the p-glycoprotein inhibitor is selected from the group consisting of water soluble vitamin E and polyethylene glycols.

21. Use according to any one of Claims 18 to 20 wherein the p-glycoprotein inhibitor is water soluble vitamin E or is selected from the group consisting of PEG 400, PEG 1000, PEG 1450, PEG 4600 and PEG 8000.

22. Use according to any one of Claims 18 to 21 wherein the p-glycoprotein inhibitor is water soluble vitamin E or PEG 1000.

23. Use of a composition according to any one of Claims 18 to 22 for the manufacture of a medicament for treating allergic reactions.

## Patentansprüche

1. Kombination zur Verstärkung der Bioverfügbarkeit eines Piperidinoalkanol-Antihistamins der folgenden Formel I 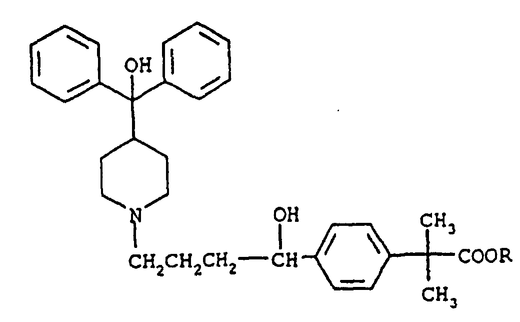 worin R Wasserstoff oder C₁-C₆-Alkyl ist,
oder eines pharmazeutisch verträglichen Salzes oder eines einzelnen optischen Isomeren davon, umfassend eine Antihistamin-wirksame Menge des Piperidinoalkanol-Antihistamins und eine effektive p-Glykoproteininhibierende Menge eines p-Glykoproteininhibitors in einer Dosierungsform, die für eine Co-Administration geeignet ist.

2. Kombination nach Anspruch 1, wobei das Antihistamin Fexofenadin oder ein pharmazeutisch verträgliches Salz davon ist.

3. Kombination nach Anspruch 1 oder 2, wobei der p-Glykoproteininhibitor aus der Gruppe, bestehend aus wasserlöslichem Vitamin E und Polyethylenglykolen, ausgewählt ist.

4. Kombination nach einem der Ansprüche 1 bis 3, wobei der p-Glykoproteininhibitor wasserlösliches Vitamin E ist oder aus der Gruppe, bestehend aus PEG 400, PEG 1000, PEG 1450, PEG 4600 und PEG 8000, ausgewählt ist.

5. Kombination nach Anspruch 4, wobei der p-Glykoproteininhibitor wasserlösliches Vitamin E oder PEG 1000 ist.

6. Kombination nach einem der Ansprüche 1 bis 5 zur Verwendung zur Behandlung von allergischen Reaktionen.

7. Kombination nach einem der Ansprüche 1 bis 6, wobei der p-Glykoproteininhibitor und die Verbindung der Formel I zur zeitlich abgestuften oder gleichzeitigen Verabreichung geeignet sind.

8. Kombination nach einem der Ansprüche 1 bis 7, wobei der p-Glykoproteininhibitor zur Verabreichung zu einer Zeit vor der Verabreichung der Verbindung der Formel I geeignet ist.

9. Kombination nach einem der Ansprüche 1 bis 8, wobei die Verbindung der Formel I und der p-Glykoproteininhibitor für im Wesentlichen gleichzeitige Verabreichung entweder in separater Dosierungsform oder in der gleichen oralen Dosierungsform geeignet sind.

10. Kombination nach Anspruch 9, wobei die orale Dosierungsform die unmittelbare Freisetzung sowohl der Verbindung der Formel I als auch des p-Glykoproteininhibitors gewährleistet.

11. Kombination nach Anspruch 9, wobei die orale Dosierungsform die verzögerte Freisetzung sowohl der Verbindung der Formel I als auch des p-Glykoproteininhibitors allein als auch Beider gewährleistet.

12. Kombination nach Anspruch 11, wobei die Dosierungsform eine Mehrfach-Kompresssionstablette ist.

13. Pharmazeutische Zusammensetzung, umfassend eine Antihistamin-wirksame Menge eines Piperidinoalkanol-Antihistamins der folgenden Formel I 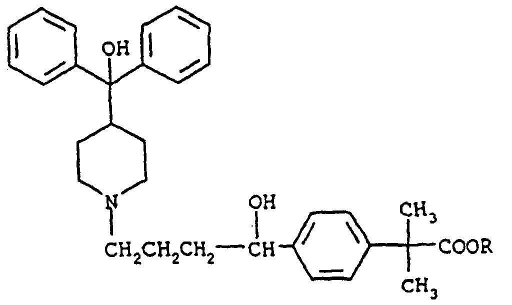 worin R Wasserstoff oder C₁-C₆-Alkyl ist,
oder ein pharmazeutisch verträgliches Salz oder ein einzelnes optisches Isomeres davon, und eine effektive p-Glykoprotein-inhibierende Menge eines p-Glykoproteininhibitors.

14. Zusammensetzung nach Anspruch 13, wobei das Antihistamin Fexofenadin oder ein pharmazeutisch verträgliches Salz davon ist.

15. Zusammensetzung nach Anspruch 13 oder 14, wobei der p-Glykoproteininhibitor aus der Gruppe, bestehend aus wasserlöslichem Vitamin E und Polyethylenglykolen, ausgewählt ist.

16. Zusammensetzung nach einem der Ansprüche 13 bis 15, wobei der p-Glykoproteininhibitor wasserlösliches Vitamin E ist oder aus der Gruppe, bestehend aus PEG 400, PEG 1000, PEG 1450, PEG 4600 und PEG 8000, ausgewählt ist.

17. Zusammensetzung nach einem der Ansprüche 13 bis 16, wobei der p-Glykoproteininhibitor wasserlösliches Vitamin E oder PEG 1000 ist.

18. Verwendung einer Zusammensetzung zur Herstellung eines Medikaments zur Erhöhung der Bioverfügbarkeit eines Piperidinoalkanol-Antihistamins der folgenden Formel I 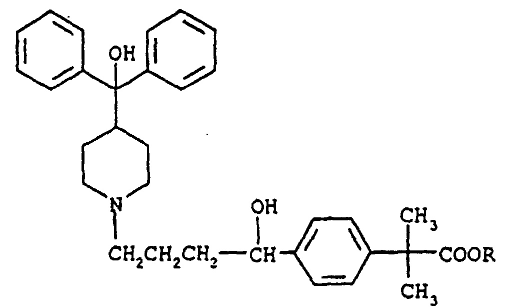 worin R Wasserstoff oder C₁-C₆-Alkyl ist,
oder eines pharmazeutisch verträglichen Salzes oder eines einzelnen optischen Isomeren davon, wobei die Zusammensetzung eine Antihistamin-wirksame Menge des Piperidinoalkanol-Antihistamins und eine wirksame p-Glykoproteininhibierende Menge eines p-Glykoproteininhibitors umfasst.

19. Verwendung nach Anspruch 18, wobei das Antihistamin Fexofenadin oder ein pharmazeutisch verträgliches Salz davon ist.

20. Verwendung nach Anspruch 18 oder 19, wobei der p-Glykoproteininhibitor aus der Gruppe, bestehend aus wasserlöslichem Vitamin E und Polyethylenglykolen, ausgewählt ist.

21. Verwendung nach einem der Ansprüche 18 bis 20, wobei der p-Glykoproteininhibitor wasserlösliches Vitamin E ist oder aus der Gruppe, bestehend aus PEG 400, PEG 1000, PEG 1450, PEG 4600 und PEG 8000, ausgewählt ist.

22. Verwendung nach einem der Ansprüche 18 bis 21, wobei der p-Glykoproteininhibitor wasserlösliches Vitamin E oder PEG 1000 ist.

23. Verwendung einer Zusammensetzung nach einem der Ansprüche 18 bis 22 zur Herstellung eines Medikaments zur Behandlung von allergischen Reaktionen.

## Revendications

1. Association permettant de renforcer la biodisponibilité d'une pipéridinoalcanol antihistamine de formule I suivante : 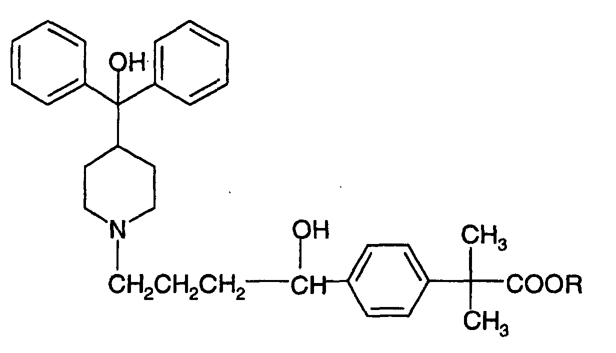 dans laquelle R est l'hydrogène ou un groupe alkyle en C₁-C₆,
ou d'un sel pharmaceutiquement acceptable ou d'un isomère optique individuel de celle-ci, qui comprend une quantité antihistaminique efficace de ladite pipéridinoalcanol antihistamine et une quantité inhibitrice de la p-glycoprotéine efficace d'un inhibiteur de la p-glycoprotéine, sous une forme galénique appropriée à la co-administration.

2. Association selon la revendication 1, dans laquelle l'antihistamine est la fexofénadine, ou un sel pharmaceutiquement acceptable de celle-ci.

3. Association selon la revendication 1 ou 2, dans laquelle l'inhibiteur de la p-glycoprotéine est sélectionné dans le groupe comprenant la vitamine E soluble dans l'eau et des polyéthylène glycols.

4. Association selon l'une quelconque des revendications 1 à 3, dans laquelle l'inhibiteur de la p-glycoprotéine'est la vitamine E soluble dans l'eau ou est sélectionné dans le groupe comprenant le PEG 400, le PEG 1000, le PEG 1450, le PEG 4600 et le PEG 8000.

5. Association selon la revendication 4, dans laquelle l'inhibiteur de la p-glycoprotéine est la vitamine E soluble dans l'eau ou le PEG 1000.

6. Association selon l'une quelconque des revendications 1 à 5, destinée à une utilisation dans le traitement de réactions allergiques.

7. Association selon l'une quelconque des revendications 1 à 6, dans laquelle l'inhibiteur de la p-glycoprotéine et le composé de formule I sont appropriés pour être administrés à des moments différents ou de manière concomitante.

8. Association selon l'une quelconque des revendications 1 à 7, dans laquelle l'inhibiteur de la p-glycoprotéine est approprié pour être administré à un moment qui précède l'administration du composé de formule I.

9. Association selon l'une quelconque des revendications 1 à 8, dans laquelle le composé de formule I et l'inhibiteur de la p-glycoprotéine sont appropriés à une administration essentiellement concomitante, soit sous une forme galénique séparée, soit sous la même forme galénique orale.

10. Association selon la revendication 9, dans laquelle la forme galénique orale fournit une libération immédiate tant du composé de formule I que de l'inhibiteur de la p-glycoprotéine.

11. Association selon la revendication 9, dans laquelle la forme galénique orale fournit une libération prolongée de l'un ou des deux parmi le composé de formule I et l'inhibiteur de la p-glycoprotéine.

12. Association selon la revendication 11, dans laquelle la forme galénique est un comprimé de compression multiple.

13. Composition pharmaceutique comprenant une quantité antihistaminique efficace d'une pipéridinoalcanol antihistamine de formule I suivante : 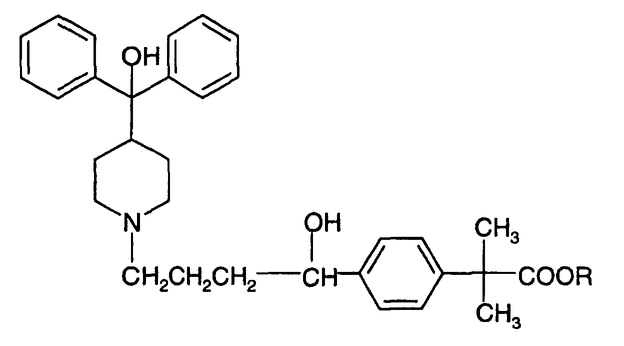 dans laquelle R est l'hydrogène ou un groupe alkyle en C₁-C₆,
ou d'un sel pharmaceutiquement acceptable ou d'un isomère optique individuel de celle-ci, et une quantité inhibitrice de la p-glycoprotéine efficace d'un inhibiteur de la p-glycoprotéine.

14. Composition selon la revendication 13, dans laquelle l'antihistamine est la fexofénadine, ou un sel pharmaceutiquement acceptable de celle-ci.

15. Composition selon la revendication 13 ou 14, dans laquelle l'inhibiteur de la p-glycoprotéine est sélectionné dans le groupe comprenant la vitamine E soluble dans l'eau et des polyéthylène glycols.

16. Composition selon l'une quelconque des revendications 13 à 15, dans laquelle l'inhibiteur de la p-glycoprotéine est la vitamine E soluble dans l'eau ou est sélectionné dans le groupe comprenant le PEG 400, le PEG 1000, le PEG 1450, le PEG 4600 et le PEG 8000.

17. Composition selon l'une quelconque des revendications 13 à 16, dans laquelle l'inhibiteur de la p-glycoprotéine est la vitamine E soluble dans l'eau ou le PEG 1000.

18. Utilisation d'une composition dans la fabrication d'un médicament pour renforcer la biodisponibilité d'une pipéridinoalcanol antihistamine de formule I suivante : 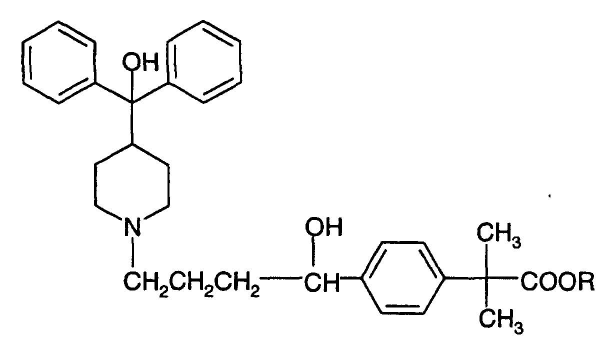 dans laquelle R est l'hydrogène ou un groupe alkyle en C₁-C₆,
ou d'un sel pharmaceutiquement acceptable ou d'un isomère optique individuel de celle-ci, dans laquelle ladite composition comprend une quantité antihistaminique efficace de ladite pipéridinoalcanol antihistamine et une quantité inhibitrice de la p-glycoprotéine efficace d'un inhibiteur de la p-glycoprotéine.

19. Utilisation selon la revendication 18, dans laquelle l'antihistamine est la fexofénadine, ou un sel pharmaceutiquement acceptable de celle-ci.

20. Utilisation selon la revendication 18 ou 19, dans laquelle l'inhibiteur de la p-glycoprotéine est sélectionné dans le groupe comprenant la vitamine E soluble dans l'eau et des polyéthylène glycols.

21. Utilisation selon l'une quelconque des revendications 18 à 20, dans laquelle l'inhibiteur de la p-glycoprotéine est la vitamine E soluble dans l'eau ou est sélectionné dans l'ensemble comprenant le PEG 400, le PEG 1000, le PEG 1450, le PEG 4600 et le PEG 8000.

22. Utilisation selon l'une quelconque des revendications 18 à 21, dans laquelle l'inhibiteur de la p-glycoprotéine est la vitamine E soluble dans l'eau ou le PEG 1000.

23. Utilisation d'une composition selon l'une quelconque des revendications 18 à 22, pour la fabrication d'un médicament destiné à traiter des réactions allergiques.
